# EUROPEAN PATENT SPECIFICATION

(11) **EP 2 229 349 B1**
(45) Date of publication and mention of the grant of the patent: **26.12.2012**
(21) Application number: 08859901.4
(22) Date of filing: 17.11.2008
(51) Int. Cl.: B01J 29/48, B01J 29/90, B01J 38/04, B01J 38/10, B01J 38/12, B01J 38/30, C07C 5/10, C07C 13/18, C07C 13/50, C07C 2/76

(54) **CONTINUOUS PROCESS FOR OXYGEN-FREE CONVERSION OF METHANE**
KONTINUIERLICHES VERFAHREN ZUR SAUERSTOFFFREIEN UMWANDLUNG VON METHAN
PROCEDE CONTINU DE CONVERSION DE METHANE EN L'ABSENCE D'OXYGENE

(30) Priority: 05.12.2007 US 992417 P
(43) Date of publication of application: 22.09.2010
(73) Proprietor: Dow Global Technologies LLC, Midland, MI 48674 (US)
(72) Inventor: GULOTTY, Robert, J., Jr., Midland, MI 48640 (US); PELATI, Joseph, E., Houston, TX 77007 (US); PRUNIER, Arthur, R., Jr., Midland, MI 48640 (US); SCHWEIZER, Albert, E., Jr., Port St. Lucie, FL 34986 (US)
(74) Representative: Raynor, John
(86) International application number: PCT/US2008/083745
(87) International publication number: WO 2009/076005

(56) References cited:
- DE-A1-102005 052 094
- US-A- 4 705 908
- US-A- 5 254 765
- US-A1- 2002 072 642

## Description

### BACKGROUND OF THE INVENTION

### 1. Technical Field

In some aspects or embodiments, this invention relates to dehydroaromatization of methane to form liquid aromatic compounds and hydrogenated derivatives, and hydrogen. More particularly, it relates to a method, preferably a continuous method, for producing liquid hydrocarbons and hydrogenated derivatives from methane or feed streams containing methane.

### 2. Background of the Art

Transportable liquid hydrocarbons, such as cyclohexane and DECALIN™ (decahydronaphthalene), are important commodities for fuel and chemical use. Currently, liquid hydrocarbons are most frequently produced from crude oil-based feedstocks by a variety of processes. However, as world supplies of crude oil feedstocks decrease, there is a growing need to find alternative sources of liquid hydrocarbons.

One possible alternative source of liquid hydrocarbons is methane, a major constituent of natural gas and biogas. Because of problems associated with transportation of large volumes of natural gas, most of the natural gas produced along with oil, particularly at remote locations, is currently flared and therefore wasted. Hence, conversion of alkanes contained in natural gas directly to higher hydrocarbons is a particularly attractive method of upgrading natural gas. However, there are attendant technical difficulties that need to be overcome.

A majority of the processes for converting methane (CH₄) to liquid hydrocarbons begins with conversion of CH₄ to synthesis gas, or "syngas," a blend of hydrogen (H₂) and carbon monoxide (CO). However, production of syngas is capital- and energy-intensive. Therefore routes that do not require syngas generation are desirable.

A number of alternative processes have been proposed for converting CH₄ directly to higher hydrocarbons. In general, these methods require catalysts. One such process involves catalytic oxidative coupling of CH₄ to form olefins, followed by the catalytic conversion of the olefins to liquid hydrocarbons, including aromatic hydrocarbons. For example, U.S. Patent No. (USP) 5,336,825 discloses a two-step process for oxidative conversion of CH₄ to gasoline-range hydrocarbons comprising aromatic hydrocarbons. In the first step, CH₄ is converted to ethylene and minor amounts of three-carbon and four-carbon (C₃ and C₄) olefins in the presence of free oxygen, using a rare earth metal-promoted alkaline earth metal oxide catalyst, at a temperature between 500 degrees centigrade (ºC) and 1000ºC. The ethylene and higher olefins formed in the first step are then converted to gasoline range liquid hydrocarbons over an acidic solid catalyst containing a high silica pentasil zeolite (e.g. H-ZSM-5).

Dehydroaromatization of CH₄ via high-temperature reductive coupling has also been proposed as a route for upgrading CH₄ into higher hydrocarbons, particularly ethylene, benzene and naphthalene. For example, USP 4,727,206 discloses a process for producing liquids rich in aromatic hydrocarbons by contacting CH₄ at a temperature between 600^{º}C and 800^{º}C, in the absence of oxygen, with a catalyst composition comprising an aluminosilicate having a silica to alumina molar ratio of at least 5:1. The aluminosilicate is loaded with both gallium, or a compound thereof, and a metal selected from Group VIIB of the Periodic Table, or a compound thereof.

USP 5,026,937 discloses a process for aromatization of CH₄ which comprises the steps of passing a feed stream, which comprises over 0.5 mole percent (mol%) H₂ and 50 mol% CH₄, into a reaction zone having at least one bed of solid catalyst comprising ZSM-5 and phosphorus-containing alumina, under conversion conditions which include a temperature of 500°C to 750°C, a pressure less than (<) 10 atmospheres (atm) (1000 kilopascals (kPa)), and a gas hourly space velocity (GHSV) of 400 cubic centimeters per gram per hour (cm³g⁻¹hr⁻¹) to 7,500 cm³g⁻¹hr⁻¹. The product effluent includes CH₄, H₂, at least (≥) 3 mol% C₂ hydrocarbons, and ≥ 5 mol% C₆ to C₈ aromatic hydrocarbons. After condensation to remove a C₄+ fraction, cryogenic techniques may be used to separate the hydrogen and light hydrocarbons (such as CH₄, ethane (C₂H₆), and ethylene) in the product effluent.

USP 5,936,135 discloses a low temperature, non-oxidative process for conversion of a lower alkane, such as CH₄ or C₂H₆, to aromatic hydrocarbons. In this process, the lower alkane is mixed with a higher olefin or paraffin (e.g., propylene or butane), and the mixture is contacted with a pretreated pentasil zeolite catalyst, such as GaZSM-5, at a temperature of 300^{º}C to 600^{º}C, a GHSV of 1000 cm³g⁻¹hr⁻¹ to 100,000 cm³g⁻¹hr⁻¹ and a pressure of 1 atm to 5 atm (100 to 500 kPa). Pretreatment of the catalyst involves contacting it with a mixture of H₂ and steam at a temperature of 400^{º}C to 800^{º}C, a pressure of 1 atm to 5 atm (100 kPa to 500 kPa) and a GHSV ≥ 500 cm³g⁻¹hr⁻¹ for a period of at least 0.5 hour, and then contacting the catalyst with air or oxygen at a temperature of 400^{º}C to 800^{º}C, a GHSV ≥ 200 cm³g⁻¹hr⁻¹, and a pressure of 1 atm to 5 atm (100 kPa to 500 kPa) for ≥ 0.2 hour.

USP 6,239,057 and USP 6,426,442 disclose processes for producing higher carbon number hydrocarbons, such as benzene, from low carbon number hydrocarbons, such as CH₄, by contacting the latter with a catalyst comprising a porous support, such as ZSM-5, which has dispersed thereon rhenium and a promoter metal such as iron, cobalt, vanadium, manganese, molybdenum, tungsten or a mixture thereof. The addition of carbon monoxide or carbon dioxide to the feed is said to increase the yield of benzene and the stability of the catalyst.

USP 6,552,243 discloses a process for the non-oxidative aromatization of CH₄ using a catalyst composition comprising a metal-loaded, crystalline aluminosilicate molecular sieve. The catalyst is initially activated by treatment with a mixture of H₂ and a C₂ to C₄ alkane, preferably butane, and then the activated catalyst is contacted with a feedstream comprising at least 40 mol% CH₄ at a temperature of 600^{º}C to 800^{º}C, a pressure of < 5 atm (500 kPa), and a weight hourly space velocity (WHSV) of 0.1 hr⁻¹ to 10 hr⁻¹.

Existing proposals for conversion of CH₄ to aromatic hydrocarbons suffer from a variety of problems that limit their commercial potential. A particular problem is that the catalysts used in the conversion are quickly deactivated, often in a matter of hours, and the build-up of by-product coke requires regular down-time for removal. Catalysis is generally taught as applied in fixed bed apparatuses. Thus, these conversions cannot generally be carried out under steady state operation with desirable operational lifetimes, and capital costs may be prohibitive where a large scale process employs a swing bed system.

### SUMMARY OF THE INVENTION

In some aspects, this invention is a method for dehydroaromatizing CH₄, which method comprises contacting CH₄, in a circulating fluid bed reactor that contains a dehydroaromatization catalyst under conditions sufficient to dehydroaromatize the CH₄ and produce at least one liquid aromatic compound and hydrogen, the catalyst comprising montmorillonite, a non-zeolitic molybdenum compound such as molybdenum oxide, and at least one zeolite that comprises at least one element selected from the group consisting of chromium (Cr), molybdenum (Mo), iron (Fe), cobalt (Co), nickel (Ni), zinc (Zn), rhenium (Re), ruthenium (Ru), rhodium (Rh), palladium (Pd), osmium (Os), iridium (Ir), platinum (Pt), tungsten (W), vanadium (V), and combinations thereof.

In some aspects, dehydroaromatizing CH₄ also produces H₂ and the method further comprises regenerating at least a portion of the catalyst. The regeneration may be carried out in one or two steps. Where it is carried out in two steps, such regeneration may be accomplished by contacting the catalyst, in a circulating fluid bed reactor, first with H₂ and then with an oxygen-containing gas or material selected from a group consisting of oxygen (O₂), air, carbon dioxide (CO₂), carbon monoxide(CO), steam, or a mixture thereof.

In some aspects, this invention is a method for preparing cyclohexane, which method comprises contacting CH₄, in a continuously operating circulating fluid bed reactor that contains a catalyst which dehydroaromatizes CH₄ to produce at least benzene and subsequently hydrogenating the benzene to form cyclohexane. Similarly, other aromatic products such as decahydronaphthalene may be prepared where naphthalene results from dehydroaromatization of CH₄.

In some aspects of this invention, a natural gas feedstock that comprises CH₄ serves as an effective substitute for CH₄. Natural gas feedstocks often contain higher alkanes, such as ethane, propane, butane and pentane. These higher alkanes may be present in such feedstocks at levels < 10 wt%, based upon total feedstock weight, and may also be converted to liquid hydrocarbons.

### BRIEF DESCRIPTION OF THE DRAWINGS

FIGURE (FIG.) 1 is a schematic drawing of a reactor/regenerator apparatus useful in practicing various aspects of this invention.
FIG. 2 is a plot comparison of catalyst deactivation with successive H₂ regenerations.
FIG. 3 is a plot comparison of catalyst deactivation with successive regenerations using H₂ or O₂.

### DETAILED DESCRIPTION OF THE INVENTION

By employing a circulating fluid bed with, in certain embodiments, simultaneous regeneration, conversion of CH₄ to a product that generally includes both benzene and H₂ may be practiced with higher product yields and longer run-times than alternative process configurations such as those disclosed above.

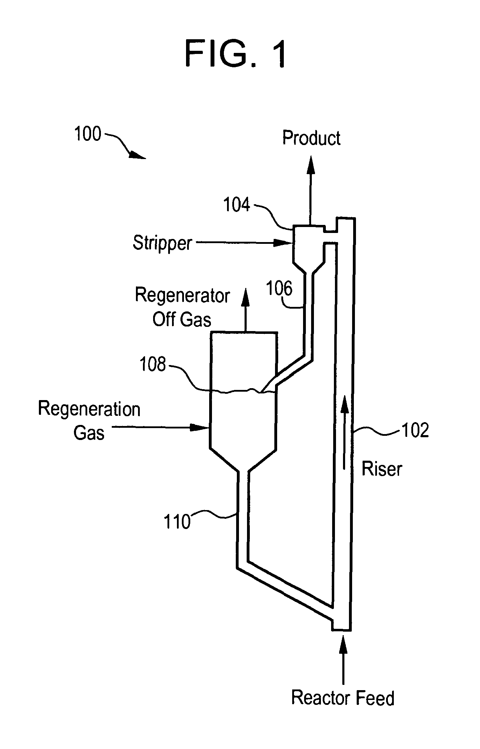

A circulating fluid bed reactor is shown schematically in FIG. 1. The reactor, generally designated 100, includes a riser 102, a separator/stripper 104, a connecting conduit or pipe 106, a regenerator 108, and a stand pipe 110, arranged in a loop around which the catalyst is continuously circulated. The catalyst particles are carried upward in the riser 102 by a stream of high velocity gas containing CH₄. The CH₄ undergoes dehydroaromatization in riser 102 to produce a product stream and the catalyst is converted to its less active form over time. The less active catalyst particles are then separated from the product stream in stripper 104 and passed through connecting conduit 106 to regenerator 108 where the deactivated catalyst is re-activated via oxidation by exposure to air or some other suitable O₂-containing gas stream. The reoxidized catalyst is then directed back into the bottom of the riser 102 by stand pipe 110.

The use of a circulating fluid bed reactor provides a number of advantages. For example, within riser 102, gas and catalyst particles approximate plug flow characteristics that enhance selectivity to benzene and H₂.

The zeolotic catalyst used in various aspects of this invention may, in some non-limiting embodiments, be selected from pentasil zeolites having, for example, ZSM-5, ZSM-8, or ZSM-11 type crystal structure. Such crystal structure consists of a large number of 5-membered oxygen-rings, i.e., the pentasil rings, which are generally believed to be more stable than other oxygen rings. ZSM-5, ZSM-8 and ZSM-11 type zeolite structures are well known and have unique shape-selective behavior. Zeolite ZSM-5 is described in greater detail in USP 3,702,886. Zeolite ZSM-8 is described in Netherlands Patent 7,014,807 and USP 3,700,585. Zeolite ZSM-11 is described in USP 3,709,979, and a ZSM-5/ZSM-11 intermediate zeolite structure is described in USP 4,229,424, both of which are incorporated herein by reference in their entireties. The term "zeolite" as used herein denotes not only microporous crystalline aluminosilicates, but also microporous crystalline galloaluminosilicates and gallosilicates.

The pentasil zeolite catalyst may be desirably selected, in some aspects, from a group consisting of gallium (Ga)-containing ZSM-5 type zolites, such as Ga-impregnated H-ZSM-5, Ga-exchanged H-ZSM-5, H-gallo-silicate of ZSM-5 type structure and H-galloaluminosilicate of ZSM-5 type structure.

The Ga-containing ZSM-5 type pentasil zeolite may contain tetrahedral aluminum (Al) and/or gallium, which is/are present in the zeolite framework or lattice, and octahedral gallium, which is not present in the zeolite framework, but rather is present in zeolite channels in close vicinity to the zeolitic protonic acid sites. The tetrahedral or framework Al and/or Ga appear(s) to be responsible for the zeolite's acid functionality and the octahedral or non-framework Ga for the zeolite's dehydrogenation functionality.

In some aspects, the pentasil zeolite is H-galloaluminosilicate of ZSM-5 type structure having a framework (tetrahedral) Si/Al and Si/Ga mole ratio of from 10:100 to 15:150, respectively. It also has non-framework (octahedral) Ga in an amount from 0.5 wt% to 5.0 wt%, based on total zeolite weight.

The catalyst may include a transition element such as Cr, Mo, Fe, Co, Ni, Zn, Re, Ru, Rh, Pd, Os, Ir, Pt, W, V, or a combination thereof. In some aspects or embodiments of this invention, a transition element is selected from a group consisting of Mo, W, Fe, V, Re, Cr, and combinations thereof. In embodiments including one or more of any of these transition elements, such may be present at levels ranging from a trace amount (greater than 0 wt%, but less than 1 wt%) to 10 wt%, preferably from 3 wt% to 6 wt%, based on total zeolite weight. In some cases the catalyst may be sulfated, i.e., include at least one sulfur (S) atom.

In non-limiting embodiments or aspects of this invention, other types of zeolites and non-zeolitic silica alumina materials, which may include the transition elements listed hereinabove, may be used to dehydroaromatize methane. These include both catalysts and catalyst promoters, such as, for example, materials with the general composition:

M^{2/n}O Al₂O₃ y SiO₂ wH₂O,

wherein y is 2 to 100, n refers to cation valence, and w represents the water contained in zeolite void spaces. M may be sodium (Na), potassium (K), magnesium (Mg), calcium (Ca), iron (Fe), or any other cation. One non-zeolitic, but still reaction-promoting, material is the clay montmorillonite, which is hydrated sodium calcium aluminum magnesium silicate hydroxide and may be represented by the formula (Na,Ca)_{0.33}(Al,Mg)₂(Si₄O₁₀)(OH)₂·nH₂O. Potassium, Fe, and other cations may be substituted, with the exact ratio of cations varying according to the source of the clay. Molybdenum oxide and other molybdenum compounds are other non-zeolites that may also be very useful in promoting dehydroaromatization. Where a molybdenum compound is selected, it may be used, in certain particular embodiments, such that the molybdenum is present in an amount from 3 to 6 wt%, based on total material weight, though higher or lower amounts may alternatively be employed. Similarly, Group Vlb elements may also be included in zeolites and/or in non-zeolites to promote dehydroaromatization.

The method of some aspects of this invention generally includes reaction conditions suitable to convert CH₄ to at least one liquid aromatic compound, such as benzene, and H₂, at a desirable or optimized conversion value.

As used herein, "liquid aromatic compound" refers to any compound that is liquid under ambient conditions and that contains at least one aromatic ring, which may be, in certain non-limiting embodiments, either partially or fully-hydrogenated. Such hydrogenation conditions may include, for example, a reaction temperature for contact of CH₄ and the catalyst (which may be a combination of more than one catalyst) ranging from 400^{º}C to 1000^{º}C, or in other non-limiting embodiments, from 600^{º}C to about 900^{º}C. It further may include a contact time, for CH₄ and the catalyst, that may range from 1 second to 30 minutes and at a GHSV ranging from 150 hr⁻¹ to 20,000 hr⁻¹. This process may be carried out in any reactor suitable for circulating and fluidizing the selected catalyst and a CH₄-containing feedstream at an elevated temperature for a desired residence or contact time, in order to permit the conversion to take place.

The method of some aspects of this invention may further include regeneration of the catalyst. As used herein, variations of the terms "regenerate" and "regeneration" refer to any treatment that restores or increases the activity of a catalyst after it has taken part in the dehydroaromatization reaction (a "used catalyst"), regardless of whether the catalyst is thereby returned to its pre-use level of activity, or to a level of activity that is somewhat less than its pre-use level, but still capable of facilitating a higher rate of reaction and/or yield of the dehydroaromatization reaction than would be achieved absent a catalyst or with the used, but not regenerated, catalyst.

Variations of catalyst regeneration may be employed. For example, in one non-limiting embodiment, there may be a temperature gradient in the riser, ranging from a riser bottom temperature of 650^{º}C to a riser top temperature of 850^{º}C. In this way, oxidized catalyst from the regenerator, which may be, for example, molybdenum oxide (MoO₂ or MoO₃) on a zeolite, may be converted to an active molybdenum carbide (Mo₂C) phase in the bottom of reactor 100. This prevents loss of the molybdenum oxide phase by evaporation from the catalyst. The activated catalyst is then used throughout the rest of the riser, to convert CH₄ to a liquid aromatic hydrocarbon (e.g. benzene).

If desired, two regenerator vessels may be connected in tandem to carry out two-step regeneration, which may be more effective than single-step regeneration. One may also use a less severe regeneration such as that using H₂ or a mixture of CO/CO₂ to achieve a low oxygen concentration. A preferred mixture of CO/CO₂ has a volumetric ratio of CO to CO₂ of 1:1. This serves to remove only the coke and does not appear to destroy the active Mo₂C phase on the catalyst. In this embodiment, the less severe regenerator (LSR) would be placed in parallel with connecting conduit 106 and feed into the bottom of reactor 100 in parallel with stand pipe 110. The residence time of the catalyst in the riser or riser/LSR may be, in certain non-limiting embodiments, from 1 minute to 30 minutes. Where H₂ is selected, it may be pure or 100 % hydrogen or it may be diluted with an inert gas such as nitrogen. A first regeneration step, using H₂, steam and/or CO₂ in one regenerator vessel, may be followed by a second regeneration step, using air, diluted air, and/or diluted O₂, or nitrous oxide (N₂O) in a second regenerator vessel.

Alternatively, regeneration may be included during the reaction itself to optimize catalyst performance. One means of doing this is to include a regeneration agent (e.g. CO, CO₂, H₂, O₂, air, steam, and combinations thereof) in the feedstream, along with CH₄. While the reaction itself tends to continuously spend catalyst, a significant portion of the catalyst will be constantly regenerating itself. Particularly good performance may be obtained by combining partial regeneration of the catalyst during the reaction by including a regenerating agent with the CH₄ feedstream, and continuous disengagement of the product effluent and used catalyst, followed by a two-step regeneration of used catalyst while the product effluent is being separated into its desirable products and by-products.

For example, a relatively large molar proportion of H₂ is produced, as may be seen in the idealized Equation 1 and Equation 2 hereinbelow. These equations describe the chemistry of benzene (C₆H₆), coke and H₂ formation:
(1) 6 CH₄ --> C₆H₆ + 9 H₂
(2) CH₄ --> C(s) + 2 H₂

The H₂ produced may be separated from higher boiling components by means of distillation, compression, membrane separation, absorption techniques and combinations thereof. The hydrogen may be used thereafter for purposes such as for fuel, e.g. to fuel furnaces that are supplying heat for the dehydroaromatization reaction. Those skilled in the art will be aware of means and methods for separating the hydrogen from the remainder of the product effluent.

The liquid aromatic hydrocarbon (e.g. C₆H₆) contained in product effluent may be, in one non-limiting embodiment, separated using any of the means and methods described for separation of the hydrogen. A portion of the C₆H₆ formed will often be hydrogenated, to a small extent, by its contact with the H₂ in the product effluent. This hydrogenation may be enhanced by routing the C₆H₆ through one or more catalytic hydrogenation units and/or hydrocrackers. For example, this approach may be useful to convert at least a portion of the C₆H₆ to cyclohexane, which has a number of potential subsequent applications and is, furthermore, easily transportable.

Other by-products that may be present in the product effluent include, e.g., ethylene, propylene, and other light olefins. These materials, again, appropriately separated by means and methods known to those skilled in the art, may be used to alkylate benzene to form corresponding alkyl aromatic products (e.g. ethyl benzene and cumene), particularly those alkyl aromatics that are liquid under ambient conditions. For these conversions, conventional technologies such as acid catalysis may be conveniently and effectively applied.

In a non-limiting embodiment or aspect, a portion of the H₂ may be used to produce alcohols via reaction with furnace CO/CO₂ mixtures or atmospheric CO₂.

Finally, at least a portion of coke produced during the reaction may be combusted to produce a CO/CO₂ mixture, which may in turn be converted to methanol by hydrogenation.

In various aspects of the method of this invention, using a circulating fluid bed reactor for primary conversion of CH₄ to form at least one liquid aromatic compound, and in certain desirable embodiments, to form C₆H₆ and H₂ in particular, may be modified and/or enhanced in a number of ways to further increase or enhance the usefulness, efficiency, performance, economics, convenience, and utility of the invention. Where the catalyst regeneration aspect is also included, the potential of such increases or enhancements is further magnified. The invention in all its aspects is thus particularly well-suited to commercial-scale production.

### EXAMPLES

### Example 1

### Preparation of JPMBZ-1 0 Catalyst (comparative)

Use an MFI-type zeolite (ZSM-5) sample (Zeolyst™-15 (CBV3024E lot# 2349-93, PQ Corporation). The number 15 refers to the sample's silica-to-alumina ratio. The zeolite sample contains organic templates. Heat the sample at 100°C/hour to a set point temperature of 500ºC and calcine it at 500°C for 4 hours. Ion exchange the zeolite with 0.5 molar (M) ammonium hydroxide (25 ml solution/gram (g) catalyst), filter it, rinse it, and then calcine it again. The pore volume measured by water addition is 0.60 ml/g. Molybdenum (6 wt% Mo, based on total catalyst weight) is added to the zeolite as a promoter by incipient wetness impregnation using an aqueous ammonium heptamolybdate solution. Dry the sample at 120°C for 4 hours, then calcine it in air at 500°C for 4 hours to provide a catalyst referred to as "JPMBZ-10." Compress the catalyst sample at 18,000 pounds per square inch gauge (psig) (124 megapascals (MPa); grind the compressed sample; and sieve the ground sample to 20 mesh (0.841 millimeter (mm) sieve opening) to 40 mesh (0.420 mm sieve opening), before loading it into a reactor for catalytic evaluation.

### Synthesis of Physmix-4 Catalyst

Prepare a montmorillonite/Mo/HZSM-5 sample ("Physmx™-4") using the preparation procedure detailed above, but use a combination of montmorillonite and Zeolyst™-15. In this preparation, dissolve 0.16 g of ammonium heptamolybdate in 1.2 milliliters (ml) of deionized water to prepare an ammonium heptamolybdate solution and use the solution to wet (fill open pores) of a 1.6 g of aliquot of montmorillonite. Add 0.61 g of molybdenum (III) oxide (Mo₂O₃) to the montmorillonite via incipient wetness, then add enough water to the montmorillonite to make it thick and stirrable. Add 6 g of Zeolyst™-15 to the stirrable montmorillonite then add more water to make it once again a thick, stirrable paste. Convert the paste to ground, sieved catalyst as detailed above before loading it into a reactor for catalytic evaluation.

### Catalyst Performance Testing

Test the catalysts in fixed bed reactors. The reactors are one-quarter inch outside diameter (5.33 mm inside diameter) stainless steel tubes that are 17 inches (43.2 centimeters (cm)) in length. Use quartz chips as fillers above and below the catalyst bed (two ml of catalyst). Introduce a gas feedstream from the top and locate the reactor tubes loaded with catalyst and quartz chips and linked to a source of gas feedstream in a cylindrical heating element. Use an externally-mounted thermocouple for heat monitoring and control. Evaluate the catalysts at 750°C and different CH₄ flow rates. At a flow rate of 20 standard cubic centimeters per minute (sccm), the GHSV is 600 hr⁻¹, and at 40 sccm, the GHSV is1200 hr⁻¹. With fresh catalyst, start a flow of gas feedstream (90 volume percent (vol%) CH4 and 10 vol% nitrogen, based on total feedstream volume) at 20 sccm. Conduct gas chromatography analysis at 30 minutes and 50 minutes after initiation. Perform an oxygen regeneration as described below.

### Catalyst Activation and Catalytic Performance Testing

Activate each of the two catalysts in a separate fixed bed reactor used for testing the catalytic performance. Use 1.0 mL of each catalyst to establish a 1.25 inch (3.2 cm) high catalyst bed. Locate each catalyst bed near the center of its reactor and fill the reactor with 20 mesh (0.841 mm sieve opening) to 40 mesh (0.420 mm sieve opening) quartz filler chips above and below the bed to hold it in place.

Apply a helium gas flow of 20 sccm to the catalyst bed and heat the bed to 750°C with a heating rate of 10°C per minute. Activate each catalysts by flowing 20 sccm of gas feedstream (90 volume percent (vol%) CH4 and 10 vol% nitrogen, based on total feedstream volume) over each catalyst at 750ºC for a period of 10 minutes. Direct effluent from the catalyst bed to a gas chromatograph to analyze effluent composition. Calculate CH₄ conversion as percentage of CH₄ remaining in the effluent using the nitrogen as an internal standard. The selectivity units are carbon mol%. Calculate mole percent of coke as the percentage of carbon not accounted for in the gas phase products. Sample the effluent at various time points, referred to as "time on stream," or TOS, from initiation of activation gas flow.

After a time period of 30-90 minutes, during which a certain amount of catalyst deactivation is shown via gas chromatography analysis, replace the gas feedstream flow with either an inert helium carrier gas flow or a regeneration gas flow. Examples 2 and 3 below present regeneration procedures.

### Catalyst Performance of JPMBZ-10 and Physmix-4.

Table 1 shows test results as taken after 30 minutes TOS. For these tests, activate and test approximately 1 g of each catalyst using a gas feedstream that is 90 vol% CH₄ and 10 vol% nitrogen (N₂), each vol% being based on total feedstream volume, a reaction temperature of 750^{º}C, and a GHSV of 1200 hr⁻¹ or 1800 hr⁻¹, as indicated.

**Table 1**

| Catalyst | JPMBZ-10 (comparative) | JPMBZ-10 (comparative) | Physmx-4 |
|---|---|---|---|
| | | | |
| Measurable (%) | | | |
| GHSV | 1200 hr⁻¹ | 1800 hr⁻¹ | 1800 hr⁻¹ |
| Methane Conversion | 13.0 | 15.0 | 13.9 |
| Benzene Selectivity | 44 | 29 | 63 |
| Ethylene Selectivity | 1.8 | 1.2 | 2.0 |
| Propylene Selectivity | n.d. | n.d. | 2.7 |
| Naphthalene Selectivity | 8.3 | 9.3 | 20.7 |
| Benzene Yield | 5.7 | 4.3 | 8.8 |

| | | | |
|---|---|---|---|
| n.d. means "not detected." Hydrogen was not measured. | | | |

### Example 2 (comparative)

### Catalyst Regeneration With Hydrogen

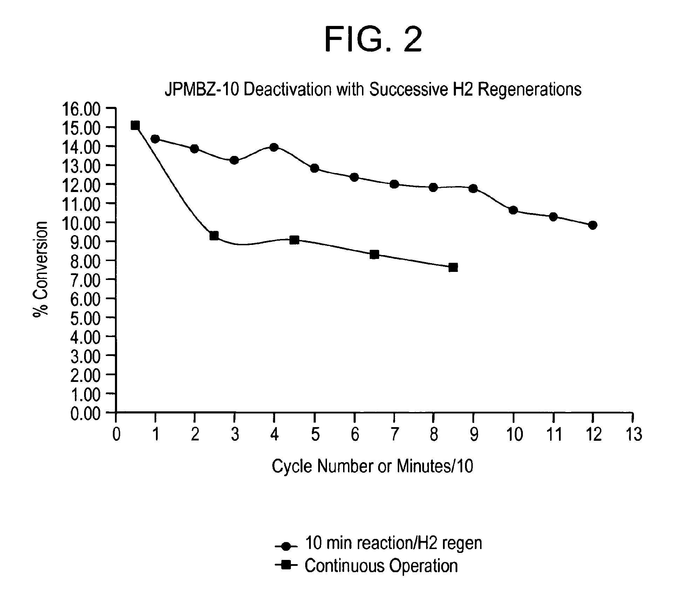

Use H₂ to provide a mild regeneration that removes hydrocarbon oligomers and soft coke. This procedure prevents build-up of large amounts of coke in general, and particularly of "hard coke", which is more difficult to remove by oxygen regeneration and may cause irreversible deactivation to the catalyst. After running a catalyst with the 90 vol% CH₄/10 vol% N₂ feedstream, switch the feedstream to a 10 vol% H₂/90 vol% N₂ feedstream, each vol% being based on total feedstream volume. After 20 minutes, restart the 90 vol% CH₄/10 vol% N₂ feedstream. Sample, via gas chromatography as detailed above, reactor effluent 10 minutes after resumption of the 90 vol% CH₄/10 vol% N₂ feedstream. Evaluate performance using JPMBZ-1 0 by comparing a series of 10 minute runs with intermittent hydrogen regenerations with a continuous flow of the 90 vol% CH₄/10 vol% N₂ feedstream and no regeneration steps. Start experiments and data collection after an O₂ regeneration, a 30 minute reaction time at 20 sccm of the 90 vol% CH₄/10 vol% N₂ feedstream, and a H₂ regeneration. The results are shown in FIGURE 2.

### Example 3 (comparative)

### Catalyst Regeneration Using Dilute Oxygen

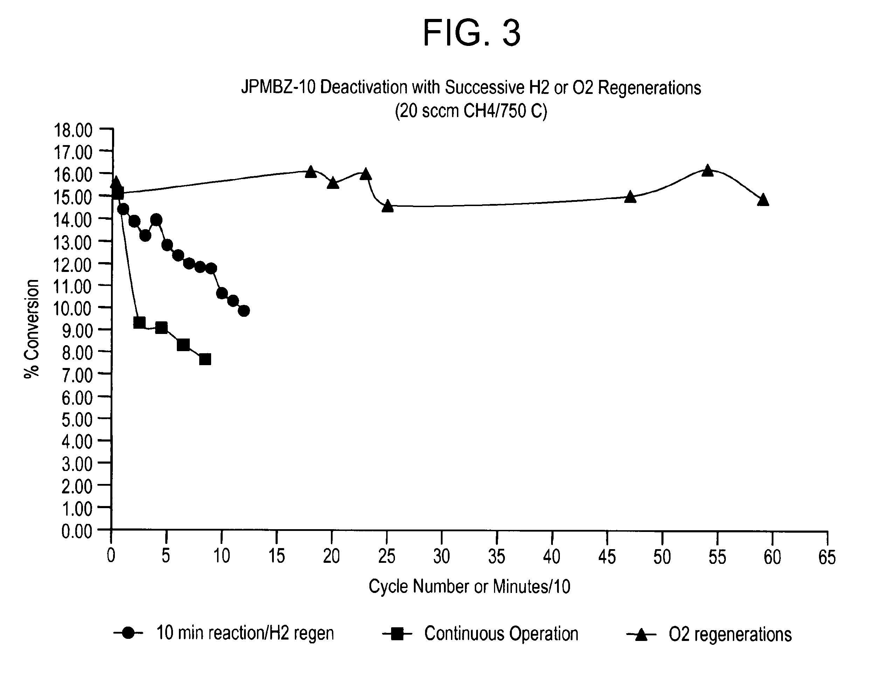

To regenerate the same catalysts as shown in Example 2, stop the 90 vol% CH₄/10 vol% N₂ feedstream and add helium to the reactor while it cools to 600°C. Run a 10 vol% O2/90 vol% N2 gas stream through the reactor, each vol% being based on total gas stream volume, at 20 sccm for 20 minutes. Purge the reactor with helium purge for at least 10 minutes, then restart the 90 vol% CH₄/10 vol% N₂ feedstream. Figure 3 shows a plot of results of multiple reaction and regeneration cycles for the JPMBZ-10 catalyst together with the data shown in FIG. 2. Reaction time minutes represents a cumulative sum of the time that the 90 vol% CH₄/10 vol% N₂ feedstream is on stream.

### Example 4 (comparative)

### Reaction Temperature and Yield

Table 2 shows yield achieved as a function of reaction temperature. There is a maximum in benzene yield at 850ºC. These data are for a 4 percent Mo/HZSM-5 catalyst prepared as described in Example 1.

**Table 2**

| Temperature (°C) | CH₄ Conversion (%) | Benzene Selectivity (%) | Benzene Yield (%) |
|---|---|---|---|
| 825 | 16.2 | 48.28 | 7.85 |
| 850 | 20.2 | 51.62 | 10.44 |
| 875 | 22.6 | 30.22 | 6.82 |

## Claims

1. A method for dehydroaromatizing methane, which method comprises contacting methane, in a circulating fluid bed reactor that contains a dehydroaromatization catalyst under conditions sufficient to dehydroaromatize methane and produce at least one liquid aromatic compound and hydrogen, the catalyst comprising montmorillonite, a non-zeolitic molybdenum compound, and at least one zeolite that comprises at least one element selected from the group consisting of chromium, molybdenum, iron, cobalt, nickel, zinc, rhenium, ruthenium, rhodium, palladium, osmium, iridium, platinum, tungsten, and vanadium.

2. The method of claim 1 further comprising a step of regenerating at least a portion of the catalyst.

3. The method of claim 2 wherein the catalyst is regenerated by contacting the catalyst with a gas selected from the group consisting of hydrogen, oxygen, carbon monoxide, carbon dioxide, air, steam, nitrous oxide and mixtures thereof.

4. The method of claim 3 wherein the conditions include a temperature from 400°C to 1000ºC.

5. The method of claim 2, claim 3 or claim 4 wherein regenerating at least a portion of the catalyst comprises two sequential steps, a) contacting the catalyst with hydrogen, and b) contacting the catalyst with a gas selected from a group consisting of oxygen, carbon monoxide, carbon dioxide, air, steam, and mixtures thereof.

6. The method of claim 1 wherein the at least one element is present in the zeolite at a concentration of from a trace amount to 10 weight percent, based on total material weight.

7. The method of any of claims 1 through 6 wherein the catalyst is sulfated.

8. The method of any of claims 1 through 7 wherein the conditions include a time period of from about 1 second to 30 minutes and a gas hourly space velocity (GHSV) of from 150 hr⁻¹ to 20,000 hr⁻¹.

9. The method of any of claims 1 through 8 wherein the at least one liquid aromatic compound is partially or fully hydrogenated.

10. The method of claim 9 wherein at least one liquid aromatic compound is benzene and the benzene is subsequently hydrogenated to form cyclohexane or the at least one liquid aromatic compound is naphthalene and the naphthalene is subsequently hydrogenated to form decahydronaphthalene.

## Patentansprüche

1. Verfahren zur Dehydroaromatisierung von Methan, wobei bei dem Verfahren Methan in einem zirkulierenden Wirbelschichtreaktor, der einen Dehydroaromatisierungskatalysator enthält, unter Bedingungen in Kontakt gebracht wird, die ausreichen, um Methan zu dehydroaromatisieren und mindestens eine flüssige aromatische Verbindung und Wasserstoff zu erzeugen, wobei der Katalysator Montmorillonit, eine nicht-zeolithische Molybdänverbindung und mindestens einen Zeolith umfasst, der mindestens ein Element aus der aus Chrom, Molybdän, Eisen, Kobalt, Nickel, Zink, Rhenium, Ruthenium, Rhodium, Palladium, Osmium, Iridium, Platin, Wolfram und Vanadium bestehenden Gruppe umfasst.

2. Verfahren nach Anspruch 1, bei dem ferner mindestens ein Teil des Katalysators regeneriert wird.

3. Verfahren nach Anspruch 2, wobei der Katalysator regeneriert wird, indem der Katalysator mit einem Gas in Kontakt gebracht wird, das aus der aus Wasserstoff, Sauerstoff, Kohlenmonoxid, Kohlendioxid, Luft, Wasserdampf, Stickstoff(I)-oxid und Mischungen davon bestehenden Gruppe ausgewählt ist.

4. Verfahren nach Anspruch 3, wobei die Bedingungen eine Temperatur von 400°C bis 1000°C umfassen.

5. Verfahren nach Anspruch 2, Anspruch 3 oder Anspruch 4, wobei das Regenerieren mindestens eines Teils des Katalysators zwei aufeinanderfolgende Schritte umfasst, nämlich a) Inkontaktbringen des Katalysators mit Wasserstoff und b) Inkontaktbringen des Katalysators mit einem Gas, das aus einer aus Sauerstoff, Kohlenmonoxid, Kohlendioxid, Luft, Wasserdampf und Mischungen davon bestehenden Gruppe ausgewählt ist.

6. Verfahren nach Anspruch 1, wobei das mindestens eine Element in dem Zeolith in einer Konzentration von einer Spurenmenge bis zu 10 Gew.-%, bezogen auf das Gesamtgewicht des Materials, vorliegt.

7. Verfahren nach einem der Ansprüche 1 bis 6, wobei der Katalysator sulfatiert ist.

8. Verfahren nach einem der Ansprüche 1 bis 7, wobei die Bedingungen einen Zeitraum von etwa 1 Sekunde bis 30 Minuten und eine stündliche Raumgeschwindigkeit des Gases (GHSV) von 150 h⁻¹ bis 20.000 h⁻¹ umfassen.

9. Verfahren nach einem der Ansprüche 1 bis 8, wobei die mindestens eine flüssige aromatische Verbindung partiell oder vollständig hydriert ist.

10. Verfahren nach Anspruch 9, wobei mindestens eine flüssige aromatische Verbindung Benzol ist und das Benzol anschließend zu Cyclohexan hydriert wird und die mindestens eine flüssige aromatische Verbindung Naphthalin ist und das Naphthalin anschließend hydriert wird, um Decahydronaphthalin zu bilden.

## Revendications

1. Procédé de déshydrogénation-aromatisation du méthane, lequel procédé comporte le fait de mettre du méthane en contact avec un catalyseur de déshydrogénation-aromatisation contenu dans un réacteur à lit fluidisé circulant, dans des conditions appropriées pour effectuer la déshydrogénation-aromatisation du méthane et produire au moins un composé aromatique liquide et de l'hydrogène, et dans lequel le catalyseur comprend de la montmorillonite, un composé du molybdène qui n'est pas une zéolithe, et au moins une zéolithe qui renferme au moins un élément choisi dans l'ensemble constitué par les chrome, molybdène, fer, cobalt, nickel, zinc, rhénium, ruthénium, rhodium, palladium, osmium, iridium, platine, tungstène et vanadium.

2. Procédé conforme à la revendication 1, qui comporte en outre le fait de régénérer au moins une partie du catalyseur.

3. Procédé conforme à la revendication 2, dans lequel on régénère le catalyseur en le mettant en contact avec un gaz choisi dans l'ensemble formé par les hydrogène, oxygène, monoxyde de carbone, dioxyde de carbone, air, vapeur d'eau et oxyde nitreux, ainsi que leurs mélanges.

4. Procédé conforme à la revendication 3, dans lequel lesdites conditions incluent une température de 400 à 1000 °C.

5. Procédé conforme à la revendication 2, 3 ou 4, dans lequel la régénération d'au moins une partie du catalyseur comporte deux étapes successives :
a) mettre le catalyseur en contact avec de l'hydrogène,
b) et mettre le catalyseur en contact avec un gaz choisi dans l'ensemble formé par les oxygène, monoxyde de carbone, dioxyde de carbone, air et vapeur d'eau, ainsi que leurs mélanges.

6. Procédé conforme à la revendication 1, dans lequel l'élément au nombre d'au moins un se trouve présent dans la zéolithe en une quantité qui peut représenter de simples traces jusqu'à 10 % du poids total du matériau.

7. Procédé conforme à l'une des revendications 1 à 6, dans lequel le catalyseur est sulfaté.

8. Procédé conforme à l'une des revendications 1 à 7, dans lequel lesdites conditions incluent un temps de séjour d'environ 1 seconde à 30 minutes et une vitesse spatiale horaire des gaz (VSHG) de 150 à 20 000 h⁻¹.

9. Procédé conforme à l'une des revendications 1 à 8, dans lequel le composé aromatique liquide au nombre d'au moins un est partiellement ou totalement hydrogéné.

10. Procédé conforme à la revendication 9, dans lequel le composé aromatique liquide au nombre d'au moins un est du benzène et ce benzène est ensuite hydrogéné pour donner du cyclohexane, ou bien le composé aromatique liquide au nombre d'au moins un est du naphtalène et ce naphtalène est ensuite hydrogéné pour donner du décahydronaphtalène.
